# EUROPEAN PATENT APPLICATION

(11) **EP 3 640 311 A1**
(43) Date of publication of application: **22.04.2020**
(21) Application number: 18818314.9
(22) Date of filing: 11.06.2018
(51) Int. Cl.: C09J 201/02, C08F 20/30, C09J 7/10, C09J 7/30, C09J 133/04

(54) **ADHESIVE COMPOSITION**

(30) Priority: 15.06.2017 JP 2017117596
(71) Applicant: Nippon Soda Co., Ltd., Tokyo 100-8165 (JP)
(72) Inventor: YAMATE, Taiki, Ichihara-shi Chiba 290-0045 (JP); TATEISHI, Yuichi, Ichihara-shi Chiba 290-0045 (JP)
(74) Representative: Wibbelmann, Jobst
(86) International application number: PCT/JP2018/022254
(87) International publication number: WO 2018/230507

(57) **Abstract**

An object of the present invention is to provide a novel adhesive composition that exhibits excellent adhesiveness even to materials having poor adhesiveness, such as polyolefin materials. The adhesive composition of the present invention comprises a polymer having a partial structure represented by formula [I] (wherein R¹ to R³ each independently represent an unsubstituted or substituted aryl group; R¹ and R², R² and R³, or R³ and R¹ are optionally bonded by a single bond; A represents an unsubstituted or substituted arylene group, and G represents a carbon atom, a silicon atom, or a germanium atom; and * represents a bonding position).

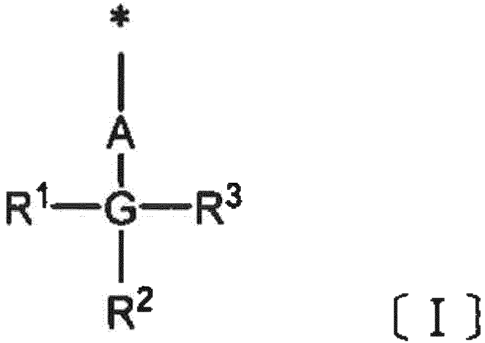

## Description

### Technical Field

The present invention relates to an adhesive composition comprising a polymer having a particular partial structure. This application claims priority to Japanese Patent Application No. 2017-117596 filed on June 15, 2017, the contents of which are incorporated herein.

### Background Art

Polyolefin materials may be produced at low cost and are easy to process and are therefore used in a variety of applications in various fields. However, the surface of polyolefin materials has the property of poor adhesiveness, and therefore there has been a need for adhesive materials suitable for polyolefin surfaces.

Patent Document 1 proposes an adhesive composition for a poorly adhesive material characterized by containing (A) a polymer having a hydrolyzable silicon-containing functional group, (B) a styrene-based (co)polymer, (C) a bisphenol type epoxy resin, and/or an epoxy resin having a polyoxyalkylene skeleton, (D) a curing catalyst for the component (A), and (E) a curing agent for the component (C). It seems that this composition may be used as an adhesive for a polyolefin material or the like.

Patent Document 2 proposes a coating agent containing a compound represented by the following formula (1) (wherein A represents a phenyl group or a naphthyl group optionally substituted by an electron-donating group; Z represents a carbon atom or a silicon atom, R² represents a hydrogen atom, a hydroxyl group, a linear or branched alkyl group, a linear or branched alkoxy group, a cyclic alkyl group, or a cyclic alkoxy group, X represents a single bond; an alkylene group that optionally comprises an oxygen atom, a sulfur atom, a selenium atom, -NR-, a divalent aliphatic ring group, an arylene group, an amide structure or a urethane structure; a divalent aliphatic ring group; or an arylene group, Y represents a polymerizable functional group, n is an integer of 2 or 3, m is an integer of 1 or 2, 1 is an integer of 0 or 1, and n + m + 1 = 4; and when n is an integer of 2 or 3, A may be the same or different). It is stated that this coating agent is excellent in adhesiveness to plastic substrates.

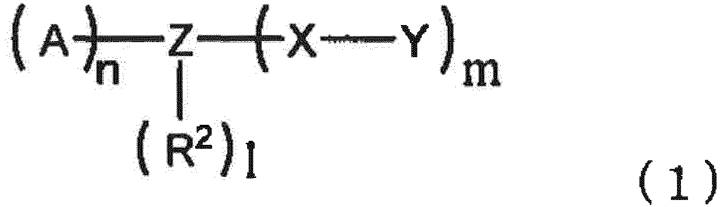

### Prior Art Documents

### Patent Documents

Patent Document 1: Japanese unexamined Patent Application Publication No. 2004-115779
Patent Document 2: WO2014/115210

### Summary of the Invention

### Object to be Solved by the Invention

An object of the present invention is to provide a novel adhesive composition that exhibits excellent adhesiveness even to materials having poor adhesiveness, such as polyolefin materials.

### Means to Solve the Object

As a result of repeated studies for achieving the above object, the present invention including the following aspects has been completed.
(1) An adhesive composition comprising a polymer having a partial structure represented by formula [I]: 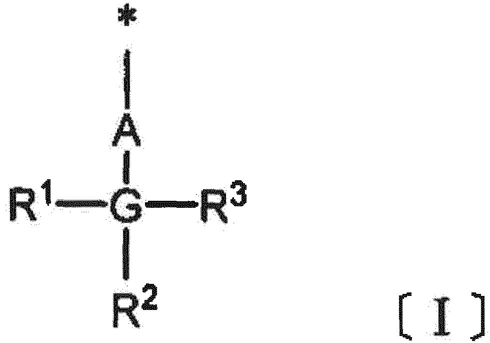 (wherein R¹ to R³ each independently represent an unsubstituted or substituted aryl group; R¹ and R², R² and R³, or R³ and R¹ are optionally bonded by a single bond; A represents an unsubstituted or substituted arylene group, and G represents a carbon atom, a silicon atom, or a germanium atom; and * represents a bonding position).
(2) The adhesive composition according to (1), wherein the polymer having the partial structure represented by formula [I] is a polymer having a repeating unit represented by formula [II]: 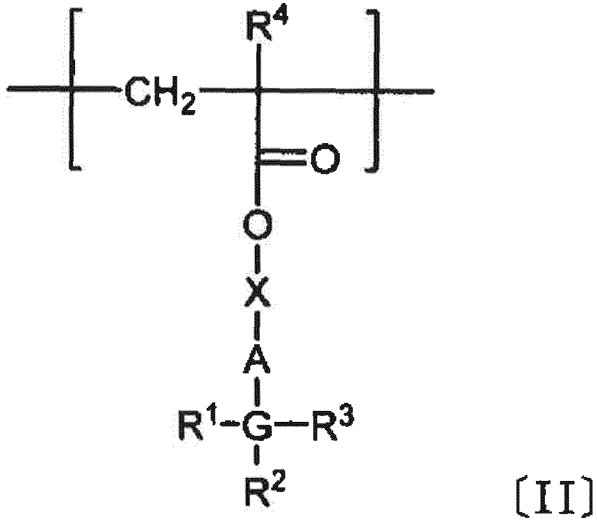 (wherein R¹ to R³ each independently represent an unsubstituted or substituted aryl group; R¹ and R², R² and R³, or R³ and R¹ are optionally bonded by a single bond; R⁴ represents a hydrogen atom or a methyl group, A represents an unsubstituted or substituted arylene group, G represents a carbon atom, a silicon atom, or a germanium atom, and X represents a single bond or a divalent linking group).
(3) The adhesive composition according to (2), wherein the polymer having the repeating unit represented by formula [II] is a polymer consisting of one or more repeating units represented by formula [II].
(4) The adhesive composition according to any one of (1) to (3), wherein the adhesive composition is for a plastic material.
(5) The adhesive composition according to any one of (1) to (3), wherein the adhesive composition is for a polyolefin material.
(6) A film formed with the adhesive composition according to any one of (1) to (5).

### Effect of the Invention

According to the present invention, an adhesive composition excellent in adhesiveness even if plastic materials such as polyolefin materials is provided. The adhesive composition may be used as an adhesive, a coating agent, or the like.

### Mode of Carrying Out the Invention

### (Adhesive Composition)

The adhesive composition of the present invention comprises a polymer having a partial structure represented by formula [I] (hereinafter sometimes described as a "polymer [I]"). It is presumed that the partial structure represented by formula [I] interacts with various materials, and therefore the adhesive composition exhibits excellent adhesiveness. The adhesive composition of the present invention may comprise other optional components as long as the effect of the present invention is not impaired.

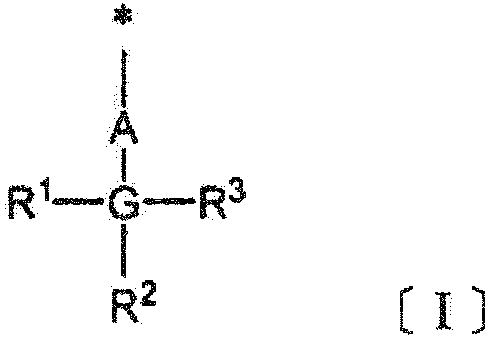

R¹ to R³ in formula [I] each independently represent an unsubstituted or substituted aryl group. As the aryl group, a phenyl group, a naphthyl group, or the like may be exemplified. Among these, a phenyl group is preferred.

As the substituent in the "substituted aryl group", a halogeno group, a hydroxy group, a C1-C6 alkyl group, a C1-C6 alkoxy group, a C1-C6 alkylthio group, a C6-C10 aryl group, a C6-C10 aryloxy group, and an amino group (a group represented by NH₂) and a C1-6 alkyl-substituted amino group may be exemplified. The "substituted aryl group" includes not only a case where an aryl group is substituted by one substituent, but also a case where an aryl group is substituted by two or more substituents.

As the halogeno group, a fluoro group, a chloro group, a bromo group, and an iodo group may be exemplified.

As the C1-C6 alkyl group, a methyl group, an ethyl group, a n-propyl group, an i-propyl group, a n-butyl group, a s-butyl group, an i-butyl group, a t-butyl group, a n-pentyl group, a n-hexyl group, or the like may be exemplified.

As the C1-C6 alkoxy group, a methoxy group, an ethoxy group, a n-propoxy group, an i-propoxy group, a n-butoxy group, a s-butoxy group, an i-butoxy group, a t-butoxy group, a n-pentoxy group, a n-hexoxy group, or the like may be exemplified.

As the C1-C6 alkylthio group, a methylthio group, an ethylthio group, a n-propylthio group, an i-propylthio group, a n-butylthio group, a s-butylthio group, an i-butylthio group, a t-butylthio group, a n-pentylthio group, a n-hexylthio group, or the like may be exemplified.

As the C6-C10 aryl group, a phenyl group, a naphthyl group, or the like may be exemplified.

As the C6-C10 aryloxy group, a phenoxy group, a naphthoxy group, or the like may be exemplified.

As the C1-6 alkyl-substituted amino group, a methylamino group, a dimethylamino group, a diethylamino group, or the like may be exemplified.

A in formula [I] represents an unsubstituted or substituted arylene group. As the arylene group, a phenylene group, a naphthylene group, or the like may be exemplified. Among these, a phenylene group is preferred.

As the substituent in the substituted arylene group, the same as the substituent in the "substituted aryl group" described for R¹ to R³ in formula [I] may be exemplified.

R¹ and R², R² and R³, or R³ and R¹ in formula [I] are optionally bonded by a single bond.

G in formula [I] represents a carbon atom, a silicon atom, or a germanium atom.

* in formula [I] represents the bonding position to the polymer main chain.

The polymer [I] also includes a polymer having two or more partial structures represented by formula [I].

The polymer [I] is not particularly limited, but is preferably a polymer obtained by polymerizing a monomer having a carbon-carbon double bond, such as a (meth)acrylic acid-based compound, a vinyl-based compound other than a (meth)acrylic acid-based compound, or a conjugated diene-based compound, and specifically, a polymer having a repeating unit represented by formula [II] (hereinafter sometimes described as a polymer [II]) may be exemplified.

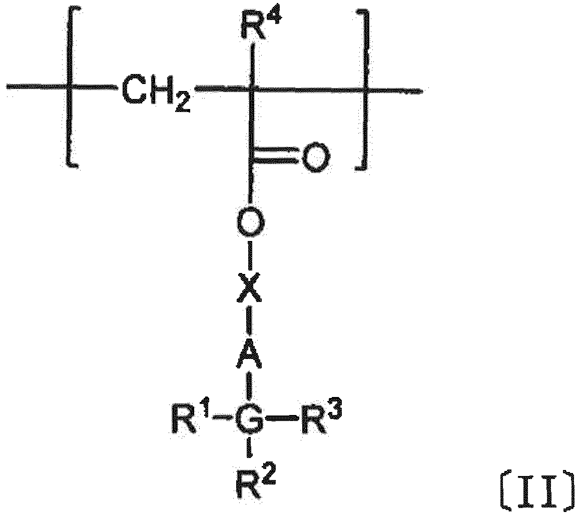

In formula [II], R¹ to R³ represent the same as R¹ to R³ in the formula [I].

In formula [II], R⁴ represents a hydrogen atom or a methyl group.

In formula [II], A represents the same as A in the formula [I].

In formula [II], G represents the same as G in the formula [I].

In formula [II], X represents a single bond or a divalent linking group. As the divalent linking group, a C1-C20 alkylene group, -O-, -S-, -NH-, -NH-C(=O)-, -NH-C(=O)-NH-, and a group made by a combination thereof, or the like may be exemplified.

The "C1-C20 alkylene group" is a divalent carbon chain, and, for example, a methylene chain, a dimethylene chain, a trimethylene chain, a methyldimethylene chain, a tetramethylene chain, a 1,2-dimethyldimethylene chain, a pentamethylene chain, a 1-methyltetramethylene chain, a 2-methyltetramethylene chain, a hexamethylene chain, an octamethylene chain, a decamethylene chain, an icosamethylene chain, or the like may be exemplified.

In the present invention, the polymer [II] also includes a polymer having two or more repeating units represented by formula [II].

For the repeating unit represented by formula [II], specifically the following repeating units may be exemplified.

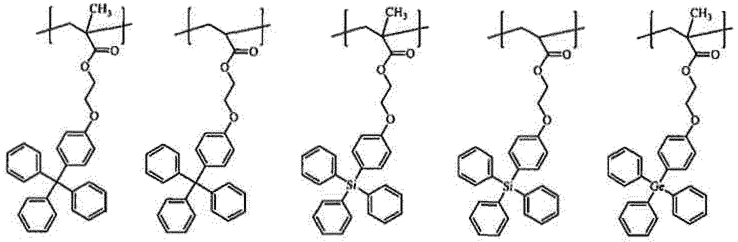

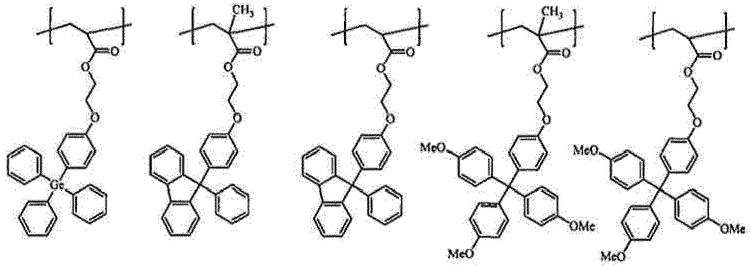

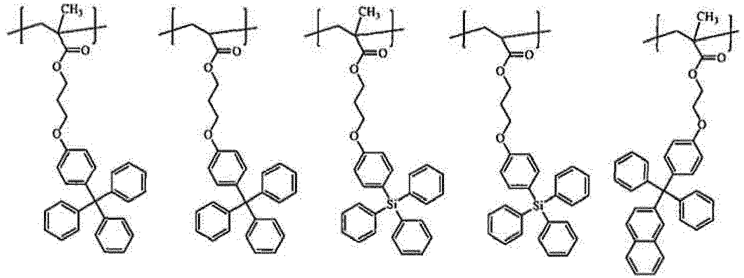

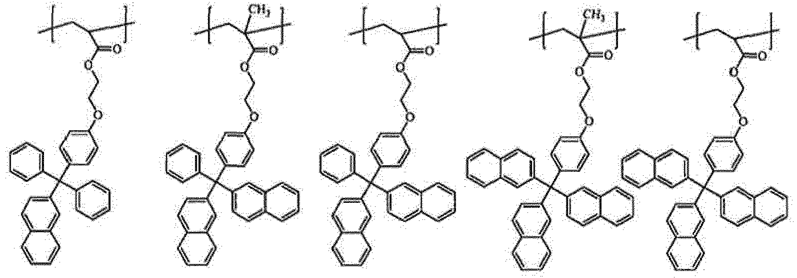

The polymer [II] may be a polymer consisting of one or more repeating units represented by formula [II], or a copolymer comprising another repeating unit other than the repeating unit represented by formula [II]. The content of another repeating unit may be 80% by weight or less, 70% by weight or less, 60% by weight or less, 50% by weight or less, 40% by weight or less, 30% by weight or less, 20% by weight or less, 10% by weight or less, 5% by weight or less, or 1% by weight or less with respect to the total weight of the polymer.

As another repeating unit in the copolymer comprising another repeating unit other than the repeating unit represented by formula [II], a repeating unit derived from a (meth)acrylate-based monomer (excluding the repeating unit represented by formula [II]), a repeating unit derived from a (meth)allyl ester-based monomer, a repeating unit derived from an aromatic vinyl-based monomer, a repeating unit derived from a conjugated diene-based monomer, or the like may be exemplified, and specifically, a (meth)acrylate-based monomer such as methyl (meth)acrylate, benzyl (meth)acrylate, phenyl (meth)acrylate, bromophenyl (meth)acrylate, ethylene glycol di(meth)acrylate, cyclohexyl (meth)acrylate, or tris(2-(meth)acryloxyethyl) isocyanate;
a (meth)allyl ester-based monomer such as diallyl phthalate, diallyl isophthalate, diallyl terephthalate, diethylene glycol bisallyl carbonate, triallyl cyanurate, or triallyl isocyanurate;
an aromatic vinyl-based monomer such as styrene, chlorostyrene, or bromostyrene; or the like is exemplified.

These compounds may be used alone or used by mixing two or more thereof.

A (meth)acrylate means an acrylate or a methacrylate, and a (meth)allyl ester means an allyl ester or a methallyl ester.

The polymer [I] and the polymer [II] used in the present invention may be terminal-modified polymers. The polymer [I] and the polymer [II] may be linear polymers, graft polymers, or star polymers. When the polymer [I] and the polymer [II] are copolymers, they may be random copolymers or block copolymers.

The number average molecular weight (Mn) of the polymer [I] and the polymer [II] used in the present invention may be in a range such as 5,000 to 500,000, 5,000 to 400,000, 5,000 to 300,000, 5,000 to 200,000, 10,000 to 150,000, 30,000 to 120,000, or 50,000 to 100,000. The molecular weight distribution (weight average molecular weight (Mw)/number average molecular weight (Mn)) may be in a range such as 1.0 to 10.0, 1.0 to 5.0, or 1.0 to 3.0. The number average molecular weight is a value obtained by converting data measured by gel permeation chromatography (GPC), based on the molecular weight of standard poly(methyl methacrylate).

The polymer [I] may be synthesized by polymerizing a monomer having the partial structure represented by formula [I]. The polymer [II] may be synthesized by polymerizing a monomer represented by the following formula [IIm]. For the polymerization method, a known polymerization method such as anionic polymerization, cationic polymerization, or radical polymerization may be used.

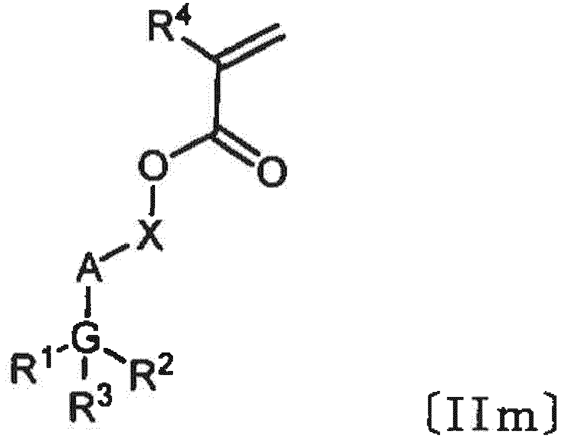

In formula [IIm], R¹ to R⁴, A, G, and X are the same as R¹ to R⁴, A, G, and X in formula [II].

The adhesive composition of the present invention may be used as an adhesive, a coating agent, a primer agent, or the like for various materials. The adhesive composition of the present invention is preferred particularly as a coating agent, an adhesive, or a primer agent for a plastic material.

As the plastic material, a polyolefin material such as a polyethylene (PE) material, a polypropylene (PP) material, or a polycycloolefin material; a polycarbonate material; an acrylic resin material; a polyimide material; a polyester material; an epoxy resin material; or the like may be exemplified. Among these, a polyolefin material is preferred, and a polycycloolefin material is more preferred.

### (Other Optional Components)

Various components may be added to the adhesive composition of the present invention according to the purpose as long as the effect of the present invention is not impaired. As such components, a binder resin, a solvent, a polymerization initiator, a flame retardant, a heat stabilizer, an antioxidant, a lubricant, an antistatic agent, an ultraviolet absorbing agent, a colorant, and a release agent, or the like is exemplified. These components may be used by combination of two or more thereof. For example, when the adhesive composition of the present invention is used as a coating agent or an adhesive, a composition to which in addition to the polymer [I] or the polymer [II], a binder resin and a solvent and the like are added is preferred.

### (Binder Resin)

As the binder resin contained in the adhesive composition of the present invention, a (meth)acrylic resin, a melamine-based resin, a urethane-based resin, an epoxy-based resin, a silicone-based resin, a polyester-based resin, a polyamic acid-based resin, a polyimide-based resin, a styrene maleic acid-based resin, a styrene maleic anhydride-based resin, or the like may be used.

In the adhesive composition of the present invention, a curable resin such as an ionizing radiation curable resin or a thermosetting resin may also be used as the binder resin. Here, the "ionizing radiation curable resin" refers to a resin cured by irradiation with any of ionizing radiation including all electromagnetic waves such as infrared rays, visible light, ultraviolet rays, X rays, and electron beams.

The above ionizing radiation curable resin or thermosetting resin is not particularly limited, but a monomer, a prepolymer, an oligomer, a polymer or the like having a vinyl group, a (meth)acryloyl group, an epoxy group, or an oxetanyl group may be used. Among them, a polyfunctional resin is preferably used. In the present invention, a "(meth)acryloyl group" means an "acryloyl group" and/or a "methacryloyl group".

As the above ionizing radiation curable resin or thermosetting resin, specifically, a monofunctional or a polyfunctional (meth)acrylate monomer, an acrylate oligomer or the like may be used. Among them, a polyfunctional acrylate or the like having two or more polymerizable unsaturated groups may be preferably used.

The above monofunctional acrylate monomer is a monomer having one (meth)acrylate group in the molecule, and, for example, tricyclodecanyl acrylate, isobornyl acrylate, tetrahydrofurfuryl acrylate, phenoxyethyl acrylate, or the like is exemplified. In the present invention, a "(meth)acrylate group" means an "acrylate group " and/or a "methacrylate group ".

The above polyfunctional acrylate monomer is a monomer having two or more and preferably two to six (meth)acrylate groups in the molecule, and, for example, tricyclodecanedimethylol diacrylate, neopentyl glycol diacrylate, hydroxypivalic acid neopentyl glycol diacrylate, dioxane glycol diacrylate, trimethylolpropane triacrylate, pentaerythritol triacrylate, pentaerythritol tetraacrylate, ditrimethylolpropane tetraacrylate, bisphenol A type polyethoxylate diacrylate, dipentaerythritol pentaacrylate, dipentaerythritol hexaacrylate, or the like is exemplified.

As the acrylate oligomer, for example, an epoxy acrylate oligomer, a polyester polyacrylate oligomer, a urethane acrylate oligomer, or the like is exemplified.

These polyfunctional or monofunctional (meth)acrylate monomers or oligomers may be used alone or used by combination of two or more thereof.

### (Polymerization Initiator)

When an ionizing radiation curable resin or a thermosetting resin is used as a binder resin, the adhesive composition of the present invention comprises a polymerization initiator. As the polymerization initiator, a photopolymerization initiator and a thermal polymerization initiator may be exemplified.

As the photopolymerization initiator that may be used in the present invention, for example, an acetophenone-based initiator such as 4-(2-hydroxyethoxy)phenyl(2-hydroxy-2-propyl)ketone [Darocur (registered trademark)-2959: manufactured by Merck]; α-hydroxy-α, α'-dimethylacetophenone [Darocur (registered trademark)-1173: manufactured by Merck]; methoxyacetophenone, or 2,2'-dimethoxy-2-phenylacetophenone [Irgacure (registered trademark) -651] ; a benzoin ether-based initiator such as benzoin ethyl ether or benzoin isopropyl ether; and in addition, a halogenated ketone, an acylphosphinoxide, an acylphosphonate, or the like may be exemplified.

The thermal polymerization initiator that may be used in the present invention includes an azo initiator and a peroxide-based initiator.

As the azo initiator, for example, azoisobutyronitrile, 1,1'-azobis(cyclohexane-1-carbonitrile), 2,2'-azobis{2-methyl-N-[1,1-bis(hydroxymethyl)ethyl]propionamide}, 2,2'-azobis[2-methyl-N-(2-hydroxyethyl)propionamide], 2,2'-azobis[2-(hydroxymethyl)propionitrile], 2,2'-azobis(2,4-dimethylvaleronitrile), 2,2'-azobis(4-methoxy-2,4-dimethylvaleronitrile), dimethyl 2,2'-azobisisobutyrate, 2,2'-azobis[2-(2-imidazolin-2-yl)propane], 2,2'-azobis{2-methyl-N-[1,1-bis(hydroxymethyl)-2-hydroxyethyl]propionamide}, or the like may be exemplified. Among these, azoisobutyronitrile is preferred in terms of cost and versatility.

As the peroxide-based initiator, for example, benzoyl peroxide, isobutyryl peroxide, cumyl peroxyoctoate, or the like may be exemplified. As the peroxide-based initiator, one which has a short thermal polymerization time and is stable as a reactive composition before polymerization may be appropriately selected.

For the blended amount of the polymerization initiator used in the adhesive composition of the present invention, the polymerization initiator is preferably blended in an amount of 0.01 to 20% by weight, and further preferably 0.1 to 10% by weight with respect to the total amount of the curable resin.

### (Solvent)

The adhesive composition of the present invention may further comprise a solvent. The solvent is not particularly limited, but for example, water; ketones such as methyl ethyl ketone, methyl isobutyl ketone, cyclohexanone, or acetylacetone; alcohols such as ethanol, isopropyl alcohol, isobutyl alcohol, n-butanol, or diacetone alcohol; ether group-containing alcohols such as ethyl cellosolve, butyl cellosolve, or propylene glycol monomethyl ether; hydroxy esters such as methyl lactate, ethyl lactate, or butyl lactate; β-keto esters such as ethyl acetoacetate, methyl acetoacetate, or butyl acetoacetate; aromatic hydrocarbons such as toluene or xylene; or the like is used. These organic solvents may be used alone or used by combination of two or more thereof.

### (Flame Retardant)

As the flame retardant, a bromine-based flame retardant usually used for a thermoplastic resin is preferred, and an inorganic flame retardant may be used in combination. As the bromine-based flame retardant, an aliphatic type, an aromatic type, a phenol type, an epoxy type, a bisphenol type, a biphenyl type, or the like is exemplified. As the inorganic flame retardant, antimony trioxide, tin oxide, molybdenum oxide, zinc borate, or the like is exemplified.

### (Heat Stabilizer)

As the heat stabilizer, a phosphorus-based heat stabilizer such as a phosphite or a phosphate may be exemplified.

As the phosphite, for example, a triester, a diester, or a monoester of phosphorous acid such as triphenyl phosphite, trisnonylphenyl phosphite, tris(2,4-di-tert-butylphenyl) phosphite, trinonyl phosphite, tridecyl phosphite, trioctyl phosphite, trioctadecyl phosphite, distearyl pentaerythritol diphosphite, tricyclohexyl phosphite, monobutyl diphenyl phosphite, monooctyl diphenyl phosphite, distearyl pentaerythritol diphosphite, bis(2,4-di-tert-butylphenyl) pentaerythritol phosphite, bis(2,6-di-tert-butyl-4-methylphenyl) pentaerythritol phosphite, or 2,2-methylenebis(4,6-di-tert-butylphenyl) octyl phosphite, or the like is exemplified.

As the phosphate, trimethyl phosphate, triethyl phosphate, tributyl phosphate, trioctyl phosphate, triphenyl phosphate, tricresyl phosphate, tris(nonylphenyl) phosphate, 2-ethylphenyl diphenyl phosphate, tetrakis(2,4-di-tert-butylphenyl)-4,4-diphenylene phosphonite, or the like is exemplified.

### (Antioxidant)

As the antioxidant, for example, a phenol-based antioxidant, a phosphorus-based antioxidant, a phosphite-based antioxidant, a thiourea-based antioxidant, or the like is exemplified.

### (Lubricant)

As the lubricant, higher fatty acids, ester waxes, polyethylene waxes, metallic soaps, or the like are exemplified.

### (Antistatic Agent)

As the antistatic agent, a fatty acid amine, a fatty acid alcohol, a fatty acid ester, a fatty acid amide, a sulfonic acid compound, or the like is exemplified.

### (Ultraviolet Absorbing Agent)

As the ultraviolet absorbing agent, a salicylic acid derivative compound, a benzophenone compound, a benzotriazole-based compound such as a benzotriazole derivative, a cyanoacrylate compound, or the like is exemplified.

### (Colorant)

As the colorant, an organic pigment, an inorganic pigment, a dye, a brightener, or the like is exemplified. As the organic pigment, for example, a phthalocyanine-based, benzimidazolone-based, azo-based, azomethineazo-based, azomethine-based, anthraquinone-based, perinone-perylene-based, indigo-thioindigo-based, dioxazine-based, quinacridone-based, isoindoline-based, or isoindolinone-based pigment or the like, or a carbon black pigment or the like is exemplified, and as the inorganic pigment, for example, an extender pigment, a titanium oxide-based pigment, an iron oxide-based pigment, a spinel pigment, or the like is exemplified. In more detail, a conventionally known pigment such as an insoluble azo pigment such as toluidine red, toluidine maroon, Hansa yellow, benzidine yellow, or pyrazolone red, a soluble azo pigment such as lithol red, Helio Bordeaux, pigment scarlet, or permanent red 2B, a phthalocyanine-based pigment such as phthalocyanine blue or phthalocyanine green, a quinacridone-based pigment such as quinacridone red or quinacridone magenta, a perylene-based pigment such as perylene red or perylene scarlet, an isoindolinone-based pigment such as isoindolinone yellow or isoindolinone orange, a pyranthrone-based pigment such as pyranthrone red or pyranthrone orange, a thioindigo-based pigment, a condensed azo pigment, a benzimidazolone-based pigment, quinophthalone yellow, nickel azo yellow, perinone orange, anthrone orange, dianthraquinonyl red, or dioxazine violet may be used. As the dye, for example, a conventionally known dye such as a direct dye, a basic dye, a cationic dye, an acidic dye, a mordant dye, an acidic mordant dye, a sulfur dye, a naphthol dye, a disperse dye, or a reactive dye may be used. As the brightener, an aluminum paste, mica, scaly iron oxide, or the like is exemplified.

### (Release Agent)

As the release agent, at least one compound selected from an aliphatic carboxylic acid, an ester consisted of an aliphatic carboxylic acid and an alcohol, an aliphatic hydrocarbon compound having a number average molecular weight of 200 to 15000, and a polysiloxane-based silicone oil may be exemplified.

As the aliphatic carboxylic acid, a saturated or unsaturated aliphatic monovalent, divalent, or trivalent carboxylic acid may be exemplified. Here, the aliphatic carboxylic acid also includes an alicyclic carboxylic acid. As specific examples of the aliphatic carboxylic acid, palmitic acid, stearic acid, caproic acid, capric acid, lauric acid, arachic acid, behenic acid, lignoceric acid, cerotic acid, melissic acid, tetrariacontanoic acid, montanic acid, adipic acid, azelaic acid, or the like may be exemplified.

As the aliphatic carboxylic acid in the ester consisted of the aliphatic carboxylic acid and the alcohol, the same as the above aliphatic carboxylic acid may be used. As the alcohol that reacts with this aliphatic carboxylic acid to form the ester, a saturated or unsaturated monohydric alcohol, a saturated or unsaturated polyhydric alcohol, or the like may be exemplified. Here, the aliphatic also includes an alicyclic compound. As specific examples of these alcohols, octanol, decanol, dodecanol, stearyl alcohol, behenyl alcohol, ethylene glycol, diethylene glycol, glycerin, pentaerythritol, 2,2-dihydroxyperfluoropropanol, neopentylene glycol, ditrimethylolpropane, dipentaerythritol, or the like may be exemplified. Ester compounds consisted of these aliphatic carboxylic acids and alcohols may contain aliphatic carboxylic acids and/or alcohols as impurities and may be mixtures of a plurality of ester compounds.

As specific examples of the ester consisted of the aliphatic carboxylic acid and the alcohol, beeswax (a mixture comprising myricyl palmitate as a main component), stearyl stearate, behenyl behenate, stearyl behenate, glycerin monopalmitate, glycerin monostearate, glycerin distearate, glycerin tristearate, pentaerythritol monopalmitate, pentaerythritol monostearate, pentaerythritol distearate, pentaerythritol tristearate, and pentaerythritol tetrastearate may be exemplified.

As the aliphatic hydrocarbon having a number average molecular weight of 200 to 15000, a liquid paraffin, a paraffin wax, a microwax, a polyethylene wax, a Fischer-Tropsch wax, or an α-olefin oligomer having 3-12 carbon atoms, or the like may be exemplified. Here, the aliphatic hydrocarbon also includes an alicyclic hydrocarbon. These hydrocarbon compounds may be partially oxidized.

As the polysiloxane-based silicone oil, for example, dimethyl silicone oil, phenyl methyl silicone oil, diphenyl silicone oil, a fluorinated alkyl silicone, or the like is exemplified. These compounds may be used alone or used by mixing two or more thereof.

### (Method for Producing Film)

The film of the present invention is not particularly limited as long as it is a film formed from the adhesive composition of the present invention.

The film of the present invention may be easily obtained by coating a substrate with the adhesive composition by various known methods and then performing drying and the like. Further, when an ionizing radiation curable resin or a thermosetting resin is used as a binder resin, the obtained film may be irradiated with active energy rays by a known method to obtain a cured film.

The substrate to which the adhesive composition is coated is not particularly limited, but is preferably a plastic substrate. As the plastic substrate, a polyolefin substrate such as a polyethylene (PE) substrate, a polypropylene (PP) substrate, or a polycycloolefin substrate; a polycarbonate substrate; an acrylic resin substrate; a polyimide substrate; a polyester substrate; an epoxy resin substrate; or the like may be exemplified. Among these, a polyolefin substrate is preferred, and a polycycloolefin substrate is more preferred.

As the method for coating the adhesive composition, for example, a bar coating method, a roll coater method, a screen method, a flexo method, a spin coating method, a dipping method, a spray method, a slide coating method, or the like may be exemplified.

Next, the present invention will be described in more detail by giving Examples. However, the present invention is not limited to the Examples.

### Examples

### [Synthesis Example 1]

### 1) Synthesis of 2-(4-Tritylphenoxy)ethyl methacrylate

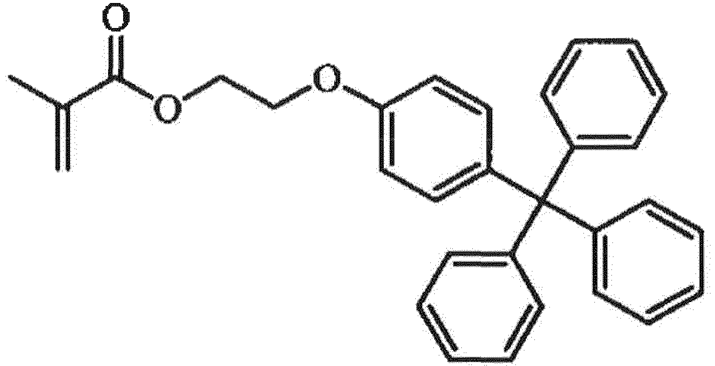

2-(4-Tritylphenoxy)ethanol (100 g, 0.26 mol) and toluene (1.5 L) were added to a 3 L four-necked flask purged with nitrogen. The mixture was stirred at room temperature for 15 min, and then methacrylic acid (27.25 g, 0.31 mol) and p-toluenesulfonic acid (25.02 g, 0.13 mol) were added. The reaction solution was heated to 110-120°C and stirred for 9 h. Then, the reaction solution was cooled to room temperature. Ethyl acetate (0.5 L) was added to the reaction solution, and the mixture was water-washed twice with distilled water. The organic layer was dehydrated with anhydrous magnesium sulfate, and then the solvent was distilled off by an evaporator. The obtained crude product was recrystallized with ethanol to obtain 2-(4-tritylphenoxy)ethyl methacrylate (81.00 g, yield 68.70%) .
¹H NMR (500 MHz, Chloroform-d₁, TMS) δ/ppm = 1.95(s, 3H), 4.20(t, 2H), 4.48(t, 2H), 5.58(s, 1H), 6.14(s, 1H), 6.81(m, 2H), 7.13-7.22(m, 19H).
¹³C NMR (125 MHz, Chloroform-d₁) δ/ppm = 18.3, 63.3, 64.4, 65.9, 113.5, 126.0, 127.6, 131.3, 132.4, 136.0, 139.6, 147.0, 156.6, 167.4.
HR-MS(TOF MS ESI, pos): 471.1909 M+Na⁺(calculated: 471.1931).

### 2) Synthesis of Poly(2-(4-tritylphenoxy)ethyl methacrylate)

The polymer was made by a thermal radical polymerization reaction using azobisisobutyronitrile (AIBN) as an initiator. 2-(4-Tritylphenoxy)ethyl methacrylate and AIBN were added to a 100 mL Schlenk tube. A stirring bar was placed, and the Schlenk tube was sealed with a three-way cock, and then a gas sampling bag containing nitrogen was mounted. The gas in the reaction vessel was degassed by a vacuum pump to be changed into nitrogen. Then, 25 mL of deoxygenated toluene was added, and the mixture was heated and stirred in an oil bath at 65°C for 24 h for a radical polymerization reaction. After the completion of the reaction, the target poly{2-(4-tritylphenoxy)ethyl methacrylate} (polymers A to E) were obtained by reprecipitation with hexane.

The number average molecular weights of the polymers A to E were adjusted by monomer (M)/AIBN ratios. The monomer (M)/AIBN ratios in the polymers, the amounts of the reagents corresponding to the ratios used, and the number average molecular weights (Mn) and molecular weight distributions (Mw/Mn) of each of the synthesized polymers are shown in Table 1.

**Table 1 Polymerization Conditions and Molecular Weight Characteristics of Polymers A to E**

| | [M] / [AIBN] | Monomer (g) | AIBN (g) | Mn (×10³) | Mw/Mn |
|---|---|---|---|---|---|
| A | 100/1 | 10.0 | 0.0366 | 23.7 | 2.04 |
| B | 200/1 | 10.0 | 0.0183 | 41.5 | 2.24 |
| C | 400/1 | 10.0 | 0.0092 | 58.9 | 1.90 |
| D | 800/1 | 10.0 | 0.0046 | 79.8 | 1.73 |
| E | 1000/1 | 10.0 | 0.0037 | 88.8 | 1.68 |

The GPC measurement conditions used for the analysis of the polymers (A to E) are shown below.

### [Apparatuses]

Sample injection apparatus: Waters 2695 Alliance
Separation columns: Shodex SB-G, SB-806HQ, SB-805HQ, SB-804HQ, and SB-803HQ
Detectors: Waters 2414 differential refractive index (RI) detector and 2998 photodiode array (PDA) detector
Column oven: column oven manufactured by Waters Corporation

### [Conditions]

Column oven: 40°C
RI detector temperature: 40°C
Mobile phase: DMF
Flow rate: 1.0 mL/min
Standard injected amount: 200 µL
PDA detector extracted wave: 254.0 nm
Quantitative calculation: standard poly(methyl methacrylate) conversion

### [Example 1]

### (Making of Adhesive Compositions)

Each of the synthesized polymers (A to E) (1.0 g) was dissolved in cyclohexanone (99.0 g) by heating to obtain adhesive compositions A to E having a solid concentration of 1 wt%.

### (Making of Coating Films)

The adhesive compositions A to E made were formed into films on various substrates having a size of 50 × 50 mm by a bar coating method so as to be roughly 300 nm thickness, thereby obtaining coating films A to E. The heating conditions were drying at 80°C for 5 min.

The substrates used are shown below.
ZEONOR Film ZF-16 (manufactured by ZEON Corporation, cycloolefin polymer (COP), 188 pm)
ZEONEX 790R (manufactured by ZEON Corporation, COP, 2 mm) APEL 6015T (manufactured by Mitsui Chemicals, Inc., cycloolefin copolymer (COC), 2 mm)

### (Evaluation of Adhesiveness)

The adhesiveness between the coating films and the polyolefin substrates was evaluated by the cross-cut tape peeling test in former JIS K5400. 11 vertical cuts and 11 horizontal cuts at intervals of 1 mm were made in each of the coating films A to E to make 100 squares. CELLOTAPE (registered trademark) was stuck and adhered to each sample by rubbing a plurality of times with the inner surface of a finger, and then the tape was peeled. Evaluation was performed by the remaining number of squares of the grid on the coating film without peeling. The results are shown in Table 2.

**Table 2. Results of Cross-Cut Tape Peeling Test**

| | Polyolefin substrates | | |
|---|---|---|---|
| Coating film | ZEONOR Film ZF-16 | ZEONEX790R | APEL6015T |
| A | 28/100 | 43/100 | 100/100 |
| B | 40/100 | 37/100 | 100/100 |
| C | 100/100 | 100/100 | 100/100 |
| D | 100/100 | 100/100 | 100/100 |
| E | 100/100 | 100/100 | 100/100 |

In Table 2, the numbers of the numerators represent the remaining number of squares of the grid on the coating film without peeling.

### [Synthesis Example 2]

### Synthesis of Random Copolymer with 2-(4-Tritylphenoxy)ethyl methacrylate/Butyl methacrylate = 75/25 (Molar Ratio)

7.51 g (16.74 mmol) of 2-(4-tritylphenoxy)ethyl methacrylate (monomer), 0.0094 g (0.06 mmol) of AIBN (product of TOKYO CHEMICAL INDUSTRY, recrystallized product), 17.80 g of toluene (Wako Pure Chemical Industries, deoxygenated grade), and 0.87 g (6.12 mmol) of butyl methacrylate (monomer) (MITSUBISHI RAYON) were added to a 50 mL two-necked flask equipped with a magnetic stirring bar, a septum rubber, a three-way cock, and a nitrogen balloon, and dissolved. Then, the gas in the flask was degassed under reduced pressure, and the solution was heated and stirred in an oil bath at 80°C. After 26 h, the heating and stirring was stopped, and the reaction solution was dropped into 800 mL of hexane to be powdered. The obtained precipitate was filtered, and dried under reduced pressure to obtain 8.77 g of a polymer F. As a result of GPC measurement, the polymer F had a number average molecular weight (Mn) of 55.5 × 10³, a weight average molecular weight (Mw) of 153 × 10³, and a molecular weight distribution (Mw/Mn) of 2.75.

The GPC measurement conditions used for the analysis of the polymer F are shown below.

### [Apparatuses]

Sample injection apparatus: 2695 Alliance manufactured by Waters Corporation
Separation columns: Shodex KF-805 L, KF-804 L, and KF-804 L
Detectors: 2414 differential refractive index (RI) detector manufactured by Waters Corporation Column oven: column oven manufactured by Waters Corporation

### [Conditions]

Column oven: 40°C
RI detector temperature: 40°C
Mobile phase: tetrahydrofuran
Flow rate: 1.0 mL/min
Standard injected amount: 100 µL
Quantitative calculation: standard polymethyl methacrylate conversion

### [Example 2]

### (Making of Adhesive Composition)

The synthesized polymer F (0.1 g) was dissolved in cyclohexanone (9.9 g) by heating to obtain an adhesive composition F having a solid concentration of 1 wt%.

### (Making of Coating Film)

A coating film F was obtained by coating the adhesive composition F made on APEL 6015T (manufactured by Mitsui Chemicals, Inc., cycloolefin copolymer (COC), 2 mm) having a size of 50 × 50 mm with a bar coating method so as to be roughly 300 nm thickness. The coated substrate was dried in an oven (120°C, 5 min) to obtain a molded body.

### (Evaluation of Adhesiveness)

The adhesiveness between the coating film F and the COC substrate was evaluated by the cross-cut tape peeling test in former JIS K5400. The result of the test was 100/100.

## Claims

1. An adhesive composition comprising a polymer having a partial structure represented by formula [I]: 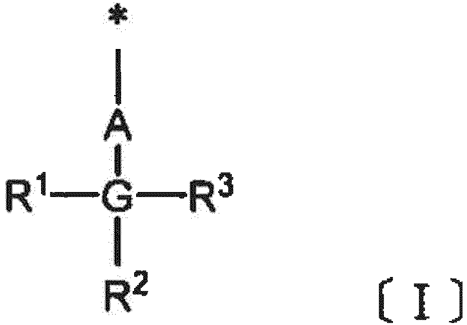 (wherein R¹ to R³ each independently represent an unsubstituted or substituted aryl group; R¹ and R², R² and R³, or R³ and R¹ are optionally bonded by a single bond; A represents an unsubstituted or substituted arylene group, and G represents a carbon atom, a silicon atom, or a germanium atom; and * represents a bonding position).

2. The adhesive composition according to claim 1, wherein the polymer having the partial structure represented by formula [I] is a polymer having a repeating unit represented by formula [II]: 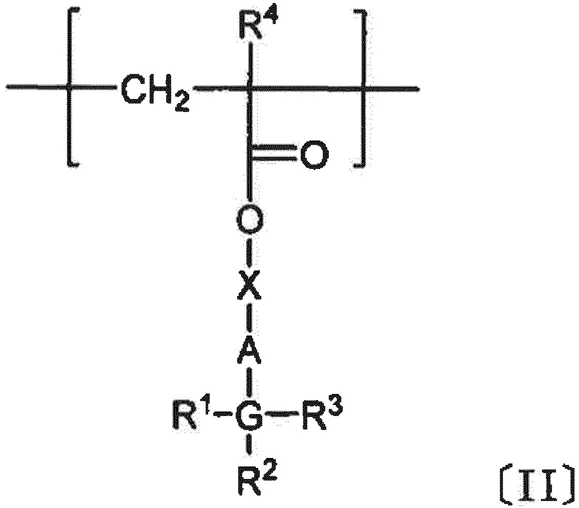 (wherein R¹ to R³ each independently represent an unsubstituted or substituted aryl group; R¹ and R², R² and R³, or R³ and R¹ are optionally bonded by a single bond; R⁴ represents a hydrogen atom or a methyl group, A represents an unsubstituted or substituted arylene group, G represents a carbon atom, a silicon atom, or a germanium atom, and X represents a single bond or a divalent linking group).

3. The adhesive composition according to claim 2, wherein the polymer having the repeating unit represented by formula [II] is a polymer consisting of one or more repeating units represented by formula [II].

4. The adhesive composition according to any one of claims 1 to 3, wherein the adhesive composition is for a plastic material.

5. The adhesive composition according to any one of claims 1 to 3, wherein the adhesive composition is for a polyolefin material.

6. A film formed with the adhesive composition according to any one of claims 1 to 5.
